# EUROPEAN PATENT SPECIFICATION

(11) **EP 2 427 142 B1**
(45) Date of publication and mention of the grant of the patent: **01.11.2017**
(21) Application number: 10719480.5
(22) Date of filing: 23.04.2010
(51) Int. Cl.: G09B 23/28, A61B 34/00, A61F 2/24

(54) **ULTRASONIC PLANNING AND GUIDANCE OF IMPLANTABLE MEDICAL DEVICES**
ULTRASCHALLPLANUNG UND -FÜHRUNG FÜR IMPLANTIERBARE MEDIZINISCHE VORRICHTUNGEN
PLANIFICATION ET GUIDAGE PAR ULTRASONS DE DISPOSITIFS MÉDICAUX IMPLANTABLES

(30) Priority: 08.05.2009 US 176501 P
(43) Date of publication of application: 14.03.2012
(73) Proprietor: Koninklijke Philips N.V., 5656 AE Eindhoven (NL)
(72) Inventor: BIANCHI, Mary, Kay, Bothell Washington 98041-3003 (US); SALGO, Ivan, Bothell Washington 98041-3003 (US)
(74) Representative: van Velzen, Maaike Mathilde
(86) International application number: PCT/US2010/032145
(87) International publication number: WO 2010/129193

(56) References cited:
- WO-A1-2010/004563
- WO-A2-02/061688
- US-A1- 2005 096 498
- ENDER J ET AL: "Value of Augmented Reality-Enhanced Transesophageal Echocardiography (TEE) for Determining Optimal Annuloplasty Ring Size During Mitral Valve Repair" THE ANNALS OF THORACIC SURGERY, ELSEVIER LNKD- DOI:10.1016/J.ATHORACSUR.2008.07.073, vol. 86, no. 5, 1 November 2008 (2008-11-01), pages 1473-1478, XP025560424 ISSN: 0003-4975 [retrieved on 2008-10-17]
- ROSEN J M ET AL: "Virtual reality and medicine: from training systems to performance machines", VIRTUAL REALITY ANNUAL INTERNATIONAL SYMPOSIUM, 1996., PROCEEDINGS OF THE IEEE 1996 SANTA CLARA, CA, USA 30 MARCH-3 APRIL 1996, LOS ALAMITOS, CA, USA,IEEE COMPUT. SOC, US, 30 March 1996 (1996-03-30), pages 5-13, XP010157041, DOI: 10.1109/VRAIS.1996.490505 ISBN: 978-0-8186-7295-8

## Description

This invention relates to medical diagnostic ultrasound systems and, in particular, to ultrasound systems which perform three dimensional image guided placement of medical devices such as prosthetic heart valves.

For the implantation of an implantable medical device such as a prosthetic heart valve there are two significant activities. The first is the planning stage in which the clinician ascertains the size or physical configuration of the implantable device which will fit properly in the anatomical implant site. A heart valve cannot be larger than the blood vessel or organ site where it is to be implanted, for instance. The second activity is the actual implantation of the device in a surgical procedure, during which the implantable device is properly located in the implant site. In many cases the device must be symmetrically located in full alignment with a vessel wall or annulus before being sutured or otherwise attached to the body, for instance. In the past, these two activities have often both been done during the surgical procedure itself. After the clinician has surgically accessed the site of the implant, the clinician will use one or more sizers which the manufacturer has provided with the implant device. The sizers are generally fabricated as nylon or plastic rings, wands, or other shapes which have one or more critical dimensions which match those of an implantable device. Heart valve manufacturers such as Medtronic, Edwards Lifesciences and St. Jude Medical provide sizers with their heart valves. Since an aortic heart valve must be the same size as the internal diameter of the aortic root and must sit on the annulus of the body's aortic valve, the sizer will exhibit a ring-like template of the size and shape of the heart valve. The ring-like template is attached to a small handle which the clinician uses to hold the ring-like template against the aortic root and valve of the patient. The clinician can then ascertain whether a heart valve with the dimensions of the sizer is a proper fit for the patient. If not, the clinician will try another sizer until one is found with template dimensions which properly fit the patient's anatomy. The clinician will then implant the heart valve of the proper size.

This important planning procedure is done at a time that is most critical for the patient and the surgeon, during the surgical procedure itself. It would be desirable to be able to do sizing for the implant before going into surgery. If sizing could be done as a pre-surgical procedure, it could be done carefully without the anxiety attendant to the surgical procedure. The properly sized implant could be obtained in advance so that it is ready for the procedure and only the implant of the proper size is in the surgical suite. Furthermore, if the implantation procedure is not open heart surgery but a minimally invasive procedure, the heart and vessels are not surgically open and available for sizing. It is then desirable to be able to size the implant device without physical access to the site of the implant.

The document of ENDER ET AL: "Value of Augmented Reality-Enhanced Transesophageal Echocardiography (TEE) for Determining Optimal Annuloplasty Ring Size During Mitral Valve Repair", THE ANNALS OF THORACIC SURGERY, ELSEVIER LNKD-DOI: 10.1016/J.ATHORACSUR.2008.07.073, vol. 86, no. 5, 1 November 2008, pages 1473-1478, XP025560424, ISSN: 003-4975 shows superimposing annuloplasty ring models on 3D echo reconstruction.

Document WO 2010/004563 A1 shows a broad viewing angle display and user interface.

The document of ROSEN J M ET AL: "Virtual reality and medicine: from training systems to performance machines", VIRTUAL REALITY ANNUAL INTERNATIONAL SYMPOSIUM, 1996., PROCEEDINGS OF THE IEEE 1996 SANTA CLARA, CA, USA 30 MARCH-3 APRIL 1996, LOS ALAMITOS, CA, USA, IEEE COMPUT. SOC, US, 30 March 1996, pages 5-13, XP010157041, DOI: 10.1109/VRAIS.1996.490505, ISBN: 978-0-8186-7295-8 shows the simulation of the impact of bullets on human tissue.

Document WO 02/061688 A2 shows modeling approaches for planning the implantation of prosthesis via surgery.

In accordance with the principles of the present invention, an ultrasound system according to claim 1 is provided. The ultrasound system includes electronic image data of an implantable device sizer, a virtual sizer. An ultrasound image is acquired of the site in the body where the device is to be implanted using 2D or 3D ultrasonic imaging. The scale of the virtual sizer image is matched to the scale of the anatomy in the ultrasound image so that the anatomy and the virtual sizer both exhibit a common scale. The virtual sizer is then manipulated against the ultrasound image to determine if the virtual sizer fits the anatomy in the ultrasound image, providing an indication of the proper implant size for the surgical procedure. The ultrasound image may be a static image, a stored loop of images, or live images. The clinician may, for example, select an image of a particular phase of the heart from a sequence (loop) of heart images to do the sizing against the dimensions of the heart during diastole or systole, as desired. When three dimensional images of the heart are used, a three dimensional virtual sizer image can be manipulated in three dimensions, just as the clinician would do in sizing during a surgical procedure, allowing assessment of implant size, orientation, and possible occlusion of other vessels. The sizing can be done against an actual anatomical ultrasound image of the implant site, or against a model of the anatomy produced from the ultrasound image data.

In the drawings:
FIGURE 1 is an illustration of a cart-borne ultrasound system.
FIGURE 2 is a block diagram of some of the subsystems of the ultrasound system of FIGURE 1.
FIGURE 3 is a block diagram of 3D beamforming in an ultrasound system of the present invention.
FIGURES 4a, 4b, and 5 illustrate automatic border detection of an anatomical border in an ultrasound image.
FIGURE 6 illustrates a 3D image of a sizer and its correspondingly sized annuloplasty ring.
FIGURES 7a-7c illustrate manipulation of a 2D image of a sizer against a model of an anatomical structure of the body.
FIGURES 8a-8e illustrate the sizing and pre-surgical deployment planning of an aortic valve for implant.
FIGURES 9a-9b illustrate sizing against a 2D MPR slice of a 3D image dataset.
FIGURES 10a-10c illustrate sizing with a 3D sizer manipulated with a 3D image.
FIGURE 11 illustrates the use of 3D ultrasound imaging to guide an implantation procedure.
FIGURE 12 illustrates the use of 3D and MPR ultrasound imaging to guide an implantation procedure.

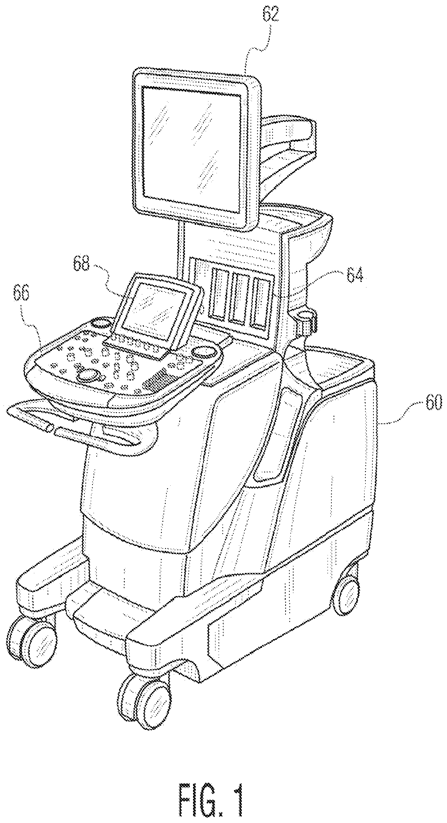

Referring first to FIGURE 1, an ultrasound system constructed in accordance with the principles of the present invention is shown. The ultrasound system includes a mainframe or chassis 60 containing most of the electronic circuitry for the system. The chassis 60 is wheel-mounted for portability. An image display 62 is mounted on the chassis 60. Different imaging probes may be plugged into three connectors 64 on the chassis. In an implementation of the present invention a matrix TEE probe which performs 3D imaging from a two-dimensional array transducer at the tip of a gastroscope located inside the esophagus or stomach is used. A suitable matrix TEE probe is described in US Pat. 6,572,547 (Miller et al.) The chassis 60 includes a control panel with a keyboard and controls, generally indicated by reference numeral 66, by which a sonographer operates the ultrasound system and enters information about the patient or the type of examination that is being conducted. At the back of the control panel 66 is a touchscreen display 68 on which programmable softkeys are displayed for specific control function. The sonographer selects a softkey on the touchscreen display 18 simply by touching the image of the softkey on the display. At the bottom of the touchscreen display is a row of buttons, the functionality of which varies in accordance with the softkey labels on the touchscreen immediately above each button.

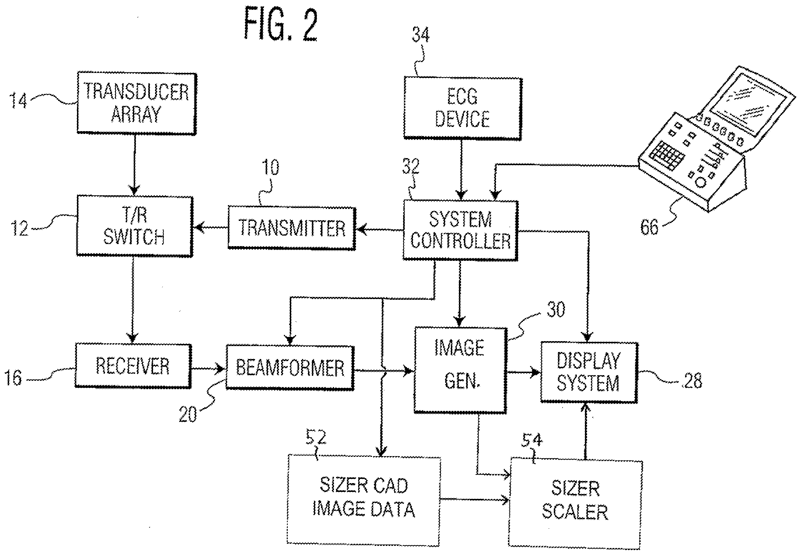

A block diagram of major elements of an ultrasound system of the present invention is shown in FIGURE 2. An ultrasound transmitter 10 is coupled through a transmit/receive (T/R) switch 12 to the transducer array 14 of the probe. Transducer array 14 is a two-dimensional array (matrix array) of transducer elements for performing three-dimensional scanning. The transducer array 14 transmits ultrasound energy into a volumetric region being imaged and receives reflected ultrasound energy, or echoes, from various structures and organs within the region. The transmitter 10 includes a transmit beamformer which controls the delay timing by which the signals applied to elements of the transducer array are timed to transmit beams of a desired steering direction and focus. By appropriately delaying the pulses applied to each transducer element by transmitter 10, the transmitter 10 transmits a focused ultrasound beam along a desired transmit scan line. The transducer array 14 is coupled through T/R switch 12 to an ultrasound receiver 16. Reflected ultrasound energy from points within the volumetric region is received by the transducer elements at different times. The transducer elements convert the received ultrasound energy to received electrical signals which are amplified by receiver 16 and supplied to a receive beamformer 20. The signals from each transducer element are individually delayed and then are summed by the beamformer 20 to provide a beamformed signal that is a representation of the reflected ultrasound energy level along points on a given receive scan line. As known in the art, the delays applied to the received signals may be varied during reception of ultrasound energy to effect dynamic focusing. The process is repeated for multiple scan lines directed throughout the volumetric region to provide signals for generating one or more images of the volumetric region as described below. Because the transducer array is two-dimensional, the receive scan lines can be steered in azimuth and in elevation to form a three-dimensional scan pattern. The beamformed signals may undergo signal processing such as filtering, Doppler processing, and image processing and buffering by an image generator 30 which produces images of different volume segments or subvolumes of a maximum volumetric region. The image data is output from image generator 30 to a display system 28 which produces a three-dimensional image of the region of interest from the image data for display on the image display 62. The display system may also construct one or more 2D image planes of the region from the three dimensional image data, a process known as multiplanar reconstruction (MPR). As discussed below, multiple different 2D images, such as three mutually orthogonal image planes, are used in one implementation of the present invention. The image generator 30 includes a scan converter which converts sector scan signals from beamformer 20 to conventional raster scan display signals. The image generator 30 also includes a volume renderer to produce three dimensional images of the imaged anatomy in the volumetric region. A system controller 32 provides overall control of the system in response to user inputs from the user controls 66 and internally stored data. The system controller 32 performs timing and control functions and typically includes a microprocessor and associated memory. The system controller is responsive to signals received from the control panel 66 and touchscreen display 68 through manual or voice control by the system user.

An ECG device 34 includes ECG electrodes attached to the patient. The ECG device 34 supplies ECG waveforms to system controller 32 for display during a cardiac exam. The ECG signals may also be used during certain exams to synchronize imaging to the patient's cardiac cycle.

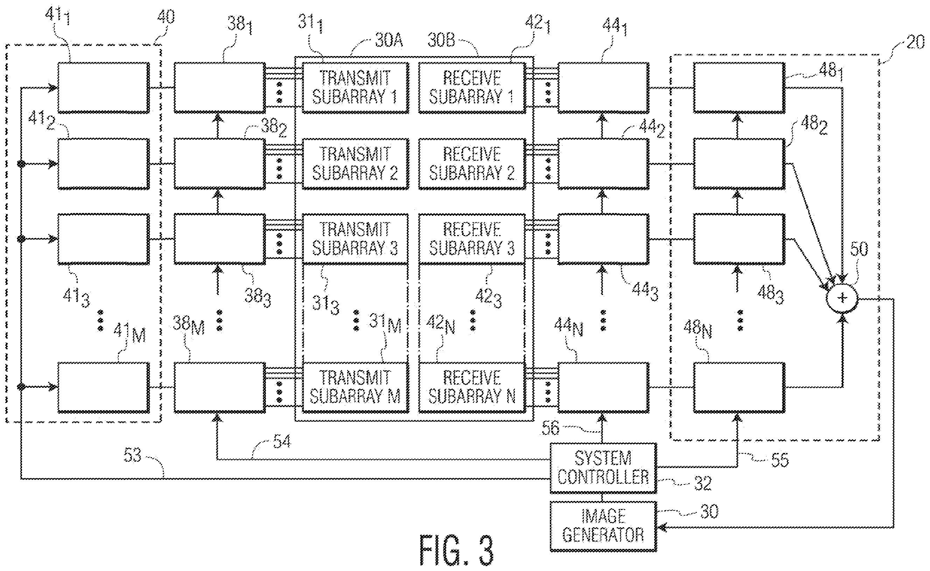

FIGURE 3 is a more detailed block diagram of an ultrasound system when operating with a matrix array for 3D imaging. The elements of the two-dimensional transducer array 14 of FIGURE 1 are divided into M transmit sub-arrays 30A connected to M intra-group transmit processors and N receive sub-arrays 30B connected to N intra-group receive processors. Specifically, transmit sub-arrays 31₁, 31₂, ... , 31_{M} are connected to intra-group transmit processors 38₁, 38₂, ... , 38_{M}, respectively, which in turn are connected to channels 41₁, 41₂, ... , 41_{M} of a transmit beamformer 40. Receive sub-arrays 42₁, 42₂, ... , 42_{N} are connected to intra-group receive processors 44₁, 44₂, ... , 44_{N}, respectively, which, in turn, are connected to processing channels 48₁, 48₂, ... , 48_{N} of a receive beamformer 20. Each intra-group transmit processor 38ᵢ includes one or more digital waveform generators that provide the transmit waveforms and one or more voltage drivers that amplify the transmit pulses to excite the connected transducer elements. Alternatively, each intra-group transmit processor 38ᵢ includes a programmable delay line receiving a signal from a conventional transmit beamformer. For example, transmit outputs from the transmitter 10 may be connected to the intra-group transmit processors instead of the transducer elements. Each intra-group receive processor 44ᵢ may include a summing delay line, or several programmable delay elements connected to a summing element (a summing junction). Each intra-group receive processor 44ᵢ delays the individual transducer signals, adds the delayed signals, and provides the summed signal to one channel 48ᵢ of receive beamformer 20. Alternatively, one intra-group receive processor provides the summed signal to several processing channels 48ᵢ of a parallel receive beamformer. The parallel receive beamformer is constructed to synthesize several receive beams simultaneously (multilines). Each intra-group receive processor 44ᵢ may also include several summing delay lines (or groups of programmable delay elements with each group connected to a summing junction) for receiving signals from several points simultaneously. The system controller 32 includes a microprocessor and an associated memory and is designed to control the operation of the ultrasound system. System controller 32 provides delay commands to the transmit beamformer channels via a bus 53 and also provides delay commands to the intra-group transmit processors via a bus 54. The delay data steers and focuses the generated transmit beams over transmit scan lines of a wedge-shaped
transmit pattern, a parallelogram-shaped transmit pattern, or other patterns including three-dimensional transmit patterns. The system controller 32 also provides delay commands to the channels of the receive beamformer via a bus 55 and delay commands to the intra-group receive processors via a bus 56. The applied relative delays control the steering and focusing of the synthesized receive beams. Each receive beamformer channel 48ᵢ includes a variable gain amplifier which controls gain as a function of received signal depth, and a delay element that delays acoustic data to achieve beam steering and dynamic focusing of the synthesized beam. A summing element 50 receives the outputs from beamformer channels 48ᵢ, 48₂, ... , 48_{N} and adds the outputs to provide the resulting beamformer signal to the image generator 30. The beamformer signals represent a receive ultrasound beam synthesized along a receive scan line. Image generator 30 constructs an image of a region probed by a multiplicity of round-trip beams synthesized over a sector-shaped pattern, a parallelogram-shaped pattern or other patterns including three-dimensional patterns. Both the transmit and receive beamformers may be analog or digital beamformers as described, for example, in U.S. Pat. Nos. 4,140,022 (Maslak); 5,469,851 (Hancock); or 5,345,426 (Lipschutz).

The system controller controls the timing of the transducer elements by employing "coarse" delay values in transmit beamformer channels 41ᵢ and "fine" delay values in intra-group transmit processors 38ᵢ. There are several ways to generate the transmit pulses for the transducer elements. A pulse generator in the transmitter 10 may provide pulse delay signals to a shift register which provides several delay values to the transmit subarrays 30A. The transmit subarrays provide high voltage pulses for driving the transmit transducer elements. Alternatively, the pulse generator may provide pulse delay signals to a delay line connected to the transmit subarrays. The delay line provides delay values to the transmit subarrays, which provide high voltage pulses for driving the transmit transducer elements. In another embodiment the transmitter may provide shaped waveform signals to the transmit subarrays 30A. Further details concerning the transmit and receive circuitry of FIGURE 3 may be found in US Pat. 6,126,602 (Savord et al.)

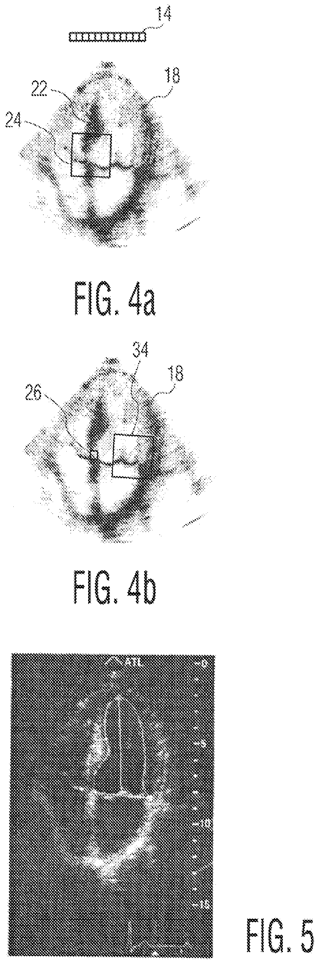

FIGURE 4a illustrates a 2D ultrasound image 18 of the heart. This ultrasound image is shown with black/white reversal of the normal appearance of an ultrasound image for clarity of illustration. In this example the transducer array 14 is opposing the apex of the heart at the top of the image. The septal wall 22 of the heart is seen extending through the center of the image. A box 24 identifies the location where the mitral valve intersects the septal wall of the heart. This point of intersection 26 can be indicated manually by a clinician viewing the image, as shown in FIGURE 4b. In FIGURE 4b a box 34 is drawn to identify the location where the mitral valve intersects the other side of the heart in the image. This point of intersection can be similarly indicated manually. There are also automated and semi-automated techniques to automatically delineate features of the heart in an ultrasound image such as the mitral valve plane and the endo- and epi-cardial borders of the myocardium. An automated technique is described in US Pat. 6,491,636 (Chenal et al.), for example, and a semi-automated method is described in US patent publication 2005/0075567 (Skyba et al.), for example. These techniques can be employed by the display system 28 in an implementation of the present invention. FIGURE 5 shows an ultrasound image in which the automated technique of Chenal et al. has been used to trace the border of the left ventricle and draw a line through the mitral valve plane.

However, a line indicating the mitral valve plane in a two dimensional image or the two points of intersection of the mitral valve with its annulus are insufficient to accurately fit or locate a mitral valve prosthesis. That is because only a single plane through the valve is shown. Even biplane imaging, where two orthogonal planes through the mitral valve are acquired, will only indicate four points of the mitral valve annulus. The mitral valve annulus cannot be assumed to be in a single plane or accurately represented by four points, as the annulus can be undulated and curved in elevation. A three dimensional ultrasound image, which can acquire a full three-dimensional data set of the mitral valve and its annulus, will depict the annulus completely and accurately. A 3D ultrasound image data set can thus be used in accordance with the present invention to produce a three dimensional image of the site of an implant, a graphical model such as a wireframe model of the implant site, or one or more selected two dimensional MPR images which can be used to gauge the fit of a prosthesis such as a heart valve prior to a valve replacement procedure.

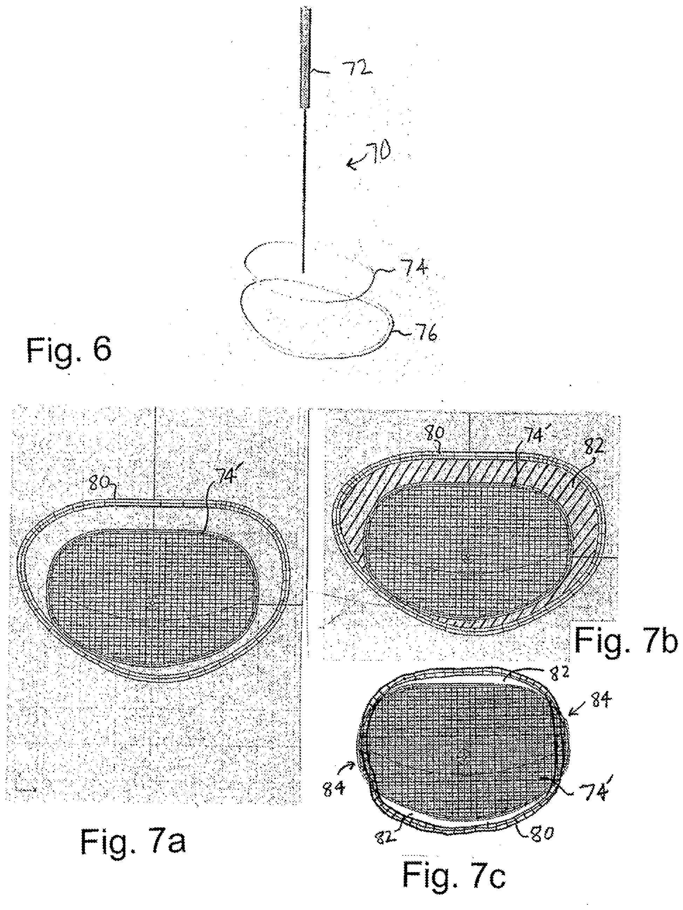

FIGURE 6 illustrates an image of a mitral valve annulus sizer 70. The sizer 70 has a handle 72 and a sizing template 74 at the end of the handle. Below the sizer is a mitral valve annuloplasty ring. The template 74 is of the size and shape required for a correspondingly sized and shaped prosthetic mitral valve and ring. During surgery the surgeon will have a variety of differently sized sizers 70 to fit to the anatomy of the patient. By trying different sizers against the patient's mitral valve annulus, the surgeon can gauge the proper size mitral valve and ring to use in replacement of the patient's mitral valve.

It would be desirable to be able to obtain this size information before surgery, so the sizing procedure could be done in advance and the proper mitral valve or ring be readied in advance of the surgical procedure. In accordance with the present invention, a digital data set of the sizer template 74 is stored in a sizer CAD image data file 52 and used to display a virtual sizer which can be manipulated with an ultrasonically developed image of the mitral valve annulus to do the sizing in advance of the procedure. Sizers are generally manufactured using a computer-aided design (CAD) procedure, which produces a digital data set of the size and shape of the sizer template. The digital data will generally define a two or three dimensional image of the sizer which can be used to display the virtual sizer. Such a CAD file image of the sizer template is manipulated against an ultrasonically-developed image of the patient's anatomy. This can be an actual ultrasound image or a model produced from the ultrasound image data and/or the traced border data such as a wire frame model as described in US Pat. 6,106,466 (Sheehan et al.) FIGURE 7a shows a virtual sizer 74' of the mitral valve template produced from CAD data of a mitral valve sizer which has been ported to the ultrasound system and stored in the sizer image data file 52. Surrounding the virtual sizer is a wire frame model of the mitral valve annulus. The wire frame model was produced from a 3D data set of the heart which included the mitral valve, and the mitral valve annulus was then delineated by border detection as described above. The clinician then manipulates a user control on the system control panel such as a trackball and joystick to move the virtual sizer 74' into alignment with the model 80 of the mitral valve annulus. Before this is done, however, the two images must be converted, if necessary to a common scale. As FIGURE 5 shows, ultrasound images are commonly delineated in centimeter increments as shown by the centimeter scale on the right side of the heart image. Thus, one of the data sets (the sizer data or the image data) can be scaled to the other so that one centimeter of the mitral valve annulus and one centimeter of the virtual sizer are both depicted in the same scale. In the implementation of FIGURE 2, this common scaling is performed by a sizer scaler 54 which is responsive to the scale of the ultrasound image form image generator 30 and the sizer image data to scale the sizer image correspondingly. With the scales so aligned, the clinician can then accurately gauge the fit of the virtual sizer and the annulus. If the particular sizer is of the wrong size, as shown in FIGURE 7a where the virtual sizer is too small, the clinician can use the CAD data and image from the file 52 for the next size virtual sizer and gauge the fit of a larger sizer. Alternatively, the data set of the original sizer can be rescaled to that of a different size virtual sizer. Different virtual sizers are used until the clinician has found the one which matches the size of the mitral valve annulus, and the clinician then knows the proper mitral valve and ring to use for the surgical procedure.

In accordance with a further aspect of the present invention, the ultrasound system indicates size and shape misalignment. In FIGURE 7b, the virtual sizer 74' is too small, leaving space 82 between the virtual sizer 74' and the mitral valve annulus 80. The display system 28 of the ultrasound system highlights this space 82 by filling it in with a distinctive color such as yellow. Pixels in the display within the wireframe model 80 which are not used in the virtual sizer 74' display are filled in with the color. When the virtual sizer completely fills the annulus, no yellow pixels will be apparent between the virtual sizer 74' and the annulus 80.

Similarly, if the virtual sizer 74' is too large, it will overlap the annulus 80 as shown at 84 in FIGURE 7c. This overlap area, where pixels of the two objects try to occupy the same pixels on the display screen, are highlighted in red. Thus, the red color tells the clinician that there is interference between the anatomy and the virtual sizer and a smaller or differently shaped virtual sizer must be used. Concurrently, if desired, the spaces 82 between the virtual sizer 74' and the annulus 80 can be filled in with the other (e.g., yellow) color. When no colors are apparent in the image display, a good fit has been obtained.

At the bottom of the ultrasound image of FIGURE 5 is an ECG trace produced by the ECG device 34 and shown with the image. The triangular carat with the vertical like extending upward indicates the phase of the heart cycle at which the ultrasound image of FIGURE 5 was acquired. Since the heart is constantly beating and thus constantly changing shape and, to a certain degree, size, the clinician can use the ECG information to obtain an ultrasound image at the phase of the heart cycle which is best used to size an implant.

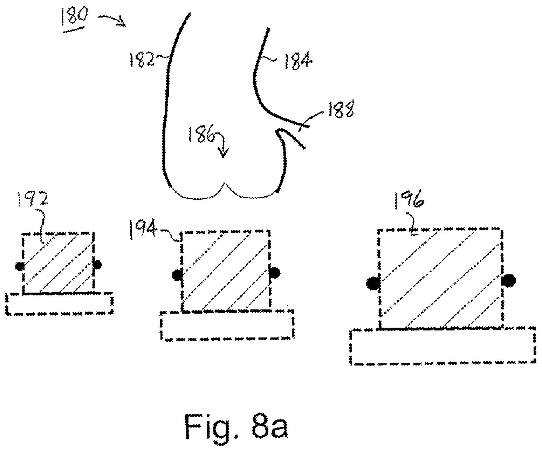

FIGURES 8a-8e illustrate the sizing process. In FIGURE 8a a cross sectional (2D) image 180 of the aorta is shown on the ultrasound display. The illustrated image shows the vessel walls 182 and 184 on opposite sides of the aorta, the coronary artery 188, and the aortic valve 186. In this procedure the aortic valve is to be replaced by an implanted valve. Shown below the aorta and aortic valve on the display screen are three correspondingly scaled virtual sizers for an aortic valve, a small sizer 192, a medium sizer 194 and a large sizer 196 for three differently sized valve replacements.

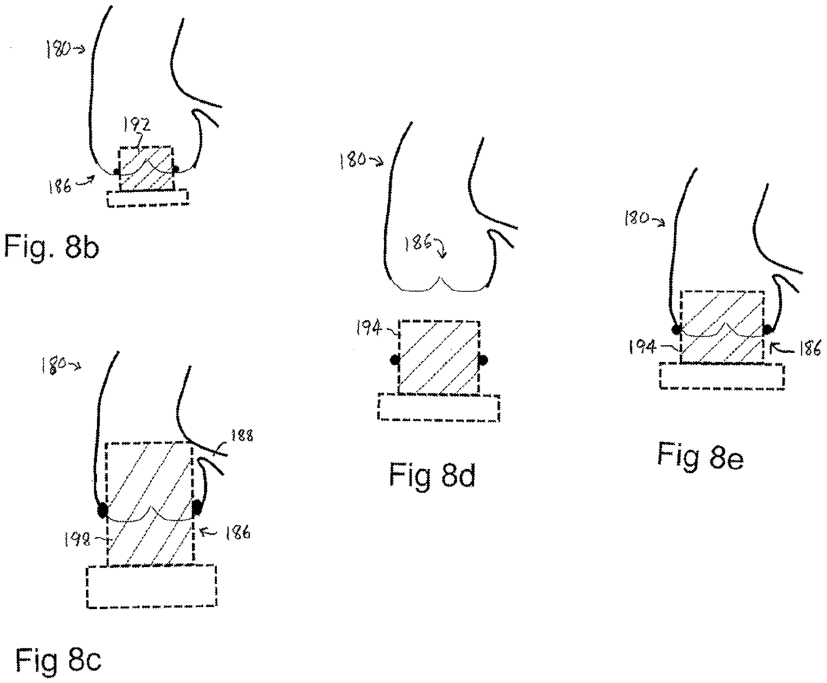

In FIGURE 8b the clinician has clicked on the small virtual sizer 192 and dragged it into the aorta 180 at the location of the aortic valve 186 which is to be replaced. As the display shows, this valve size is too small and will not adequately replace the aortic valve 186.

In FIGURE 8c another virtual sizer 198 is tried in the aorta 180 and aortic valve 186 on the display screen. This virtual sizer is seen to be of the correct diameter to fit the annulus of the aortic valve 186. However, this particular valve replacement is too long, as it is seen to obstruct the coronary artery 188.

In FIGURE 8d the clinician is manipulating the mid-sized virtual sizer 194 toward the aorta 180 and aortic valve 186 on the display screen. FIGURE 8e illustrates the display screen when the clinician has dragged the virtual sizer 194 into its desired location in the aorta and valve. The sizer is seen to fit exactly in the valve annulus and does not block the coronary artery. This sizing indicates that a replacement valve corresponding to virtual sizer 194 should be used for this valve replacement procedure, and is done in the pre-planning stage prior to surgery.

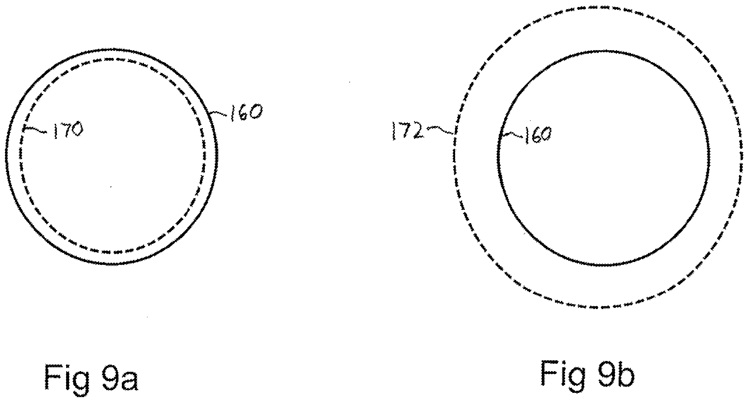

When a matrix array probe is used to acquire the ultrasound images, a three dimensional dataset can be acquired of a volume which includes the surgical site. Planar image slices can then be formed through any plane of the volume by MPR image reconstruction. A 2D image can thus be selected of the anatomy to which the implantable device is to be attached. If the anatomy is nonplanar and undulating, a number of spatially successive MPR slices can be compounded and displayed together as a thick slice image as described in international patent application publication WO2008/126015 (Thiele et al.) One such MPR image or reconstructed anatomical model 160 is illustrated in FIGURES 9a and 9b. In FIGURE 9a a virtual sizer 170 is fitted to the anatomy of the MPR image or model 160 and is seen to fit properly. In FIGURE 9b another virtual sizer 172 is seen to be too large for the anatomical opening 160.

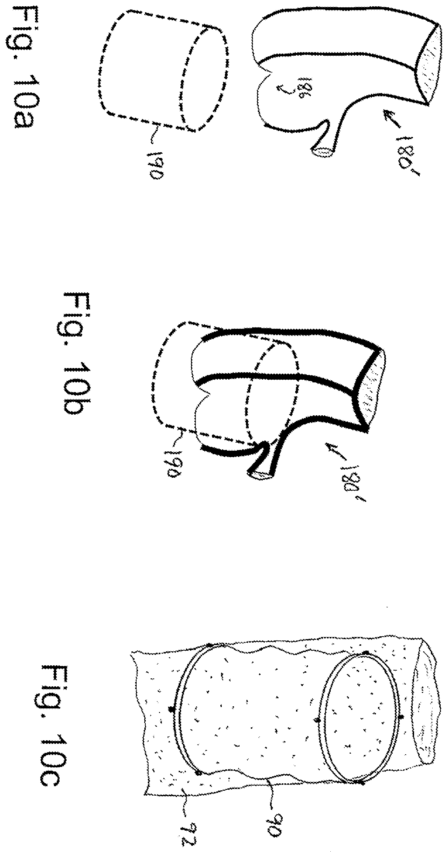

While FIGURES 7a-7c show the sizing being performed with 2D images, it can also be done in three dimensions, which is often preferable. FIGURES 10a-10c illustrate the use of three dimensional images for sizing. FIGURE 10a illustrates a display in which a scaled 3D graphic of a virtual sizer 190, which in this example is a graphical representation of the implantable device itself, is approaching a similarly scaled 3D image or model of the aorta 180' and aortic valve 186. FIGURE 10b illustrates the result after the clinician has manipulated the virtual sizer 190 into its placement location at the aortic valve. To fully assess the fit of the virtual sizer in the vessel, the opacity of the vessel and/or the virtual sizer is adjusted by the clinician. In the example of FIGURE 10b, for instance, the clinician has adjusted the display opacity of the vessel so that it is partially transparent and the fit of the virtual sizer 190 within the vessel 180' can be readily ascertained. The vessel 180' with the virtual sizer 190 inside can be tilted and rotated by kinetic parallax manipulation of the two so that the clinician can view the inserted sizer/device from different perspectives. The clinician can vary the relative transparency of the vessel and the virtual sizer/device until the clinician has thoroughly inspected the fit of the device in the vessel and is satisfied that a device of this size is appropriate for this patient.

In the display of FIGURE 10c a three dimensional ultrasound image of a blood vessel 92 is shown being aligned with a commonly scaled image of a stent or balloon device 90 as the virtual sizer. With a 3D image of both the anatomy and the implantable device, the clinician can manipulate the device image and try different size or shaped devices until a fit of the two is found. The clinician can check the fit by turning or rotating the image of the two to view the fit from all sides and angles. This procedure can similarly be aided by coloring spaces and interference regions with distinctive shading or colors.

It will be appreciated that many anatomical regions in the body have dynamic characteristics which need to be considered, as is the case of the heart. The mitral valve annulus is not static, but moves and changes shape as the heart beats. With real time ultrasound, an image sequence can be stopped at particular phases of the heart to gauge the fit of a sizer or device in the heart at those particular times of the heart cycle. The clinician may want to ascertain whether a particular annuloplasty ring works well at both end diastole and peak systole, for instance. The CAD model of the implant device can be aligned with heart images or models at those particular phases of the heart to give the clinician the assurance that the selected device works well during the complete heart cycle. It is also possible to warp or bend the virtual sizer image to better gauge the fit of the implantable device with a non-planar anatomical implant site.

A library of different device CAD image files can be installed in the ultrasound system so that the user can select the one to use for a given procedure. Alternatively, CAD image files of the devices to be used in the present procedure can be loaded, scaled to the ultrasound image (or *vice versa*), and used to determine the proper fit of a device in advance of surgery.

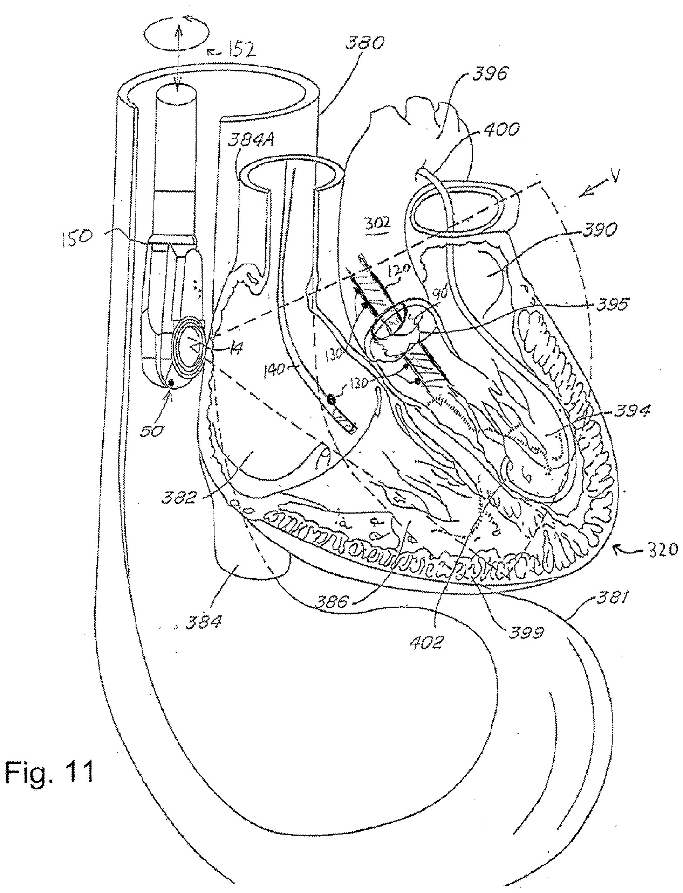

FIGURE 11 illustrates the use of 3D ultrasound to guide an actual implant procedure. In this procedure a matrix TEE probe 150 as described in the Miller et al. patent is inserted down the esophagus 380 of a patient, as indicated by arrows 152. As the arrows indicate, the TEE probe can be inserted down the esophagus, retracted back out after use, and rotated for the proper view while in the esophagus. From the esophagus or stomach the matrix transducer array 14 can view the heart as indicated by V, which is pointing to the volumetric region being viewed by the transducer. In one form of this procedure a catheter 120 with the implantable device 90' on it is inserted through an incision in the abdomen and through the myocardium at the apex 320 of the heart. In this example the implantable device 90' is an aortic valve prosthesis, and so the catheter 120 is threaded through the heart chambers to the outflow tract and the aorta 302. When the catheter has passed through the aortic valve 395 and the prosthesis 90' is aligned with valve 395, the prosthesis 90' is deployed, anchored in place, and the catheter 120 is withdrawn.

Ideally it would be desirable to observe all of this clearly in a 3D ultrasound image. However, the catheter 120 and device 90' are usually strong scatterers of ultrasound and a great deal of clutter usually surrounds their location in the image, so that their exact location often cannot be clearly perceived in the ultrasound image. In accordance with a further aspect of the present invention, this difficulty can be overcome with a surgical navigation system such as that described in US Pat. 6,785,571 (Glossop). The Glossop patent shows a field generator which produces a complex electromagnetic field through the body of the patient. Small sensors such as magnetic sensor coils produce signals which react to changes in the position and orientation of the sensors in the complex field. This enables their orientation and position in the field inside the patient to be tracked. In another implementation the tracking can be done with ultrasonic sensors as described in US Pat. 5,158,088 (Nelson et al.) In the example of FIGURE 11 a sensor 50 is located on the TEE probe to track its location, and sensors 130 are positioned on the catheter 120 ahead of and behind the device 90'. This permits the location data of the catheter from the sensors to be merged into the ultrasound image. The location of the aortic valve 395 can be located in the image before the insertion of the catheter 120 and marked in the image. The catheter 120 is inserted into the aorta until the sensors 130 are on either side of this mark. The device 90' can then be deployed where desired, even if artifacts clutter the ultrasound image and obscure the implant site.

FIGURE 11 also shows the procedure being performed by an intracardiac echocardiography (ICE) catheter 140 which observes the implant site from within another blood vessel. As in the case of the matrix TEE probe, the ICE catheter 140 includes a sensor 130 for correlating the positions of the implant catheter 120 and device 90' in the 3D image field of the ICE catheter.

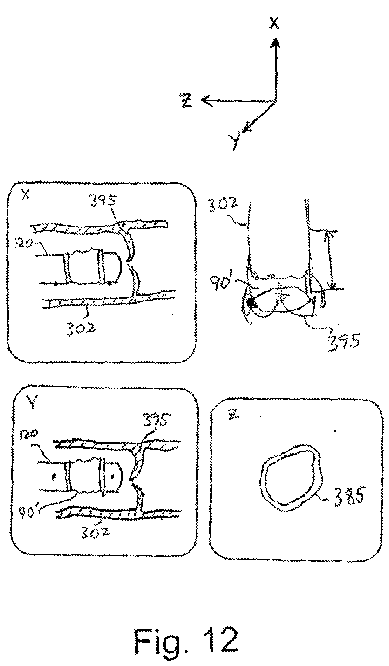

FIGURE 12 illustrates in the upper right quadrant of the drawing a 3D ultrasound image of the aorta 302 and the aortic valve 395. This can be an anatomical ultrasound image or a model of the anatomy such as a wire screen model. By selecting a point in this 3D ultrasound image, images in three planes which orthogonally intersect the point can be produced by the MPR technique as illustrated in Figs. 9-14 of the Miller et al. patent. In this example the intersection point is located at the aortic valve 395 and the three MPR planes are in the X,Y, and Z planes indicated above the 3D image. The three orthogonal planes will thus show three views of the catheter 120 and device 90' as they approach the surgical site. In this example the X and Y plane views show orthogonal cross-sectional views of the aorta 302 and aortic valve 395 as the catheter 120 approaches the valve. The Z plane in this example is through the aortic valve annulus 385 and will show the catheter when it passes through the valve. Preferably this guidance is performed with actual 3D ultrasound anatomical images. But if the clutter problem is too great, the 3D image 302 can be constructed as a model of the aorta and aortic valve prior to catheter insertion. The guidance sensors (shown as small dots in these images) will then track the position of the catheter 120 as it approaches the aortic valve 395, and an icon or other representation of the catheter (such as a model derived from the 3D CAD data of the device) can be moved toward the valve in the model 302 as the catheter 120 approaches, guided by the tracking information from the position sensors.

FIGURE 10c illustrates another beneficial use of three dimensional ultrasound in implantable device guidance, which is examining the position and orientation of the device 90 before it is deployed. Some implants such as valves and stents need to be precisely oriented in the proper position before being deployed, as once deployed their position cannot be adjusted. For instance, the device cannot be cocked or askew in the blood vessel; it must be uniformly aligned with the lumen of the vessel before being deployed. FIGURE 10c illustrates a device 90 which is cocked and askew in the vessel 92. This misalignment can be observed from all sides and angles in a 3D ultrasound image, aided by adjustment of the relative transparency of the vessel image 92 and the device, and the alignment corrected before deployment of the device 90. If needed, the alignment process can be aided by position sensors 130 on the device as described above. The ultrasound image is preferably a live 3D image, but if the clutter problem is too great, a static image or anatomical model can be acquired or produced in advance of device introduction and the adjustment then guided by sensing of the position and orientation of the device relative to the anatomy or model as taught by Glossop.

## Claims

1. An ultrasound system which is used to plan a surgical procedure with an implantable device, comprising:
an ultrasound probe (14) arranged to acquire ultrasonic echo signals from a volumetric region of the body where an implantable device is to be located;
an image generator (30) responsive to the ultrasonic echo signals from the ultrasound probe, and adapted to operate as a source of scaled anatomical ultrasound images of the site in a body where an implantable device is to be located;
a source of one or more scaled images of a sizer (70) arranged to form a virtual sizer (74') that indicates the size of the implantable device, said source comprising scaled images of virtual sizers for implantable devices of different sizes;
a scaler (54) responsive to the scaled anatomical ultrasound image from the image generator, and to the scaled image of the sizer from the source, said scaler being adapted to scale the image of the sizer to a common scale corresponding to the scaled anatomical ultrasound image;
a display system (28), responsive to the anatomical ultrasound image and the virtual sizer to highlight areas of an image where the sizer does not fit the site in the anatomical ultrasound image where the implantable device is to be located;
a display (62) adapted to display the commonly scaled anatomical ultrasound image and the virtual sizer; and
a user control (66) operable by a user to manipulate the positioning of the virtual sizer on the display with respect to the anatomy of the commonly scaled anatomical ultrasound image, said user control is further operable to try to fit the virtual sizer in the anatomy of the ultrasound image.

2. The ultrasound system of Claim 1, wherein the ultrasound images are three dimensional ultrasound images.

3. The ultrasound system of Claim 1, wherein the commonly scaled ultrasound image and the virtual sizer are three dimensional images.

4. The ultrasound system of Claim 3, wherein the three dimensional ultrasound and virtual sizer images can be tilted and rotated together in response to a user control.

5. The ultrasound system of Claim 1, wherein the image generator further comprises a border detector which traces an anatomical border of the anatomical ultrasound image.

6. The ultrasound system of Claim 1 wherein the user control is further operable by the user to vary the relative opacity or transparency of the anatomy of the ultrasound image and the virtual sizer.

7. The ultrasound system of Claim 1, wherein the virtual sizer further comprises a scaled image of an implantable device.

8. A method of ascertaining the size of an implantable device which is appropriate for the anatomy of a body comprising:
displaying a scaled ultrasound image of the anatomy of the body where the implantable device is to be located;
displaying a commonly scaled image of a virtual sizer (74') which indicates the size of an implantable device with the scaled ultrasound image of the anatomy;
displaying with highlighting the interference fit of the scaled image of the virtual sizer with the scaled ultrasound image of the anatomy; and
manipulating the virtual sizer in relation to the anatomy of the ultrasound image to ascertain whether the virtual sizer properly fits the anatomy in the ultrasound image, wherein displaying further comprises displaying a plurality of virtual sizers for implantable devices of different sizes; and
wherein manipulating further comprises selecting and manipulating one of the plurality of virtual sizers in relation to the anatomy of the ultrasound image.

9. The method of Claim 8, wherein displaying further comprises displaying a scaled three dimensional ultrasound image of the anatomy of the body.

10. The method of Claim 9, wherein manipulating further comprises rotating or tilting the virtual sizer and the anatomy together.

## Patentansprüche

1. Ultraschallsystem, das verwendet wird, um eine chirurgische Prozedur mit einer implantierbaren Vorrichtung zu planen, umfassend:
eine Ultraschallsonde (14), die dafür vorgesehen ist, Ultraschallechosignale aus einer volumetrischen Region des Körpers zu erfassen, an der die implantierbare Vorrichtung positioniert werden soll;
einen Bildgenerator (30), der auf die Ultraschallechosignale von der Ultraschallsonde reagiert und dafür ausgelegt ist, als eine Quelle von skalierten anatomischen Ultraschallbildern von derjenigen Stelle in einem Körper zu dienen, an der eine implantierbare Vorrichtung positioniert werden soll;
eine Quelle von einem oder mehreren skalierten Bildern einer Größenbestimmungsschablone (70), die dafür vorgesehen sind, eine virtuelle Größenbestimmungsschablone (74') zu bilden, die die Größe der implantierbaren Vorrichtung angibt, wobei die genannte Quelle skalierte Bilder von virtuellen Größenbestimmungsschablonen für implantierbare Vorrichtungen unterschiedlicher Größe umfasst;
einen Skalierer (54), der auf das skalierte anatomische Ultraschallbild von dem Bildgenerator und auf das skalierte Bild der Größenbestimmungsschablone von der Quelle reagiert, wobei der genannte Skalierer dafür ausgelegt ist, das Bild der Größenbestimmungsschablone auf einen gemeinsamen Maßstab entsprechend dem skalierten anatomischen Ultraschallbild zu skalieren;
ein Anzeigesystem (28), das auf das anatomische Ultraschallbild und die virtuelle Größenbestimmungsschablone reagiert, um Bereiche eines Bildes hervorzuheben, an denen die Größenbestimmungsschablone nicht für die Stelle in dem anatomischen Ultraschallbild passt, an der die implantierbare Vorrichtung positioniert werden soll;
eine Anzeige (62), die dafür ausgelegt ist, das gemeinsam skalierte anatomische Ultraschallbild und die virtuelle Größenbestimmungsschablone anzuzeigen; und
eine Benutzersteuerung (66), die durch einen Benutzer bedienbar ist, um die Positionierung der virtuellen Größenbestimmungsschablone auf der Anzeige in Bezug auf die Anatomie des gemeinsam skalierten anatomischen Ultraschallbilds zu manipulieren, wobei die genannte Benutzersteuerung weiterhin bedienbar ist, um zu versuchen, die virtuelle Größenbestimmungsschablone in die Anatomie des Ultraschallbilds einzupassen.

2. Ultraschallsystem nach Anspruch 1, wobei die Ultraschallbilder dreidimensionale Ultraschallbilder sind.

3. Ultraschallbild nach Anspruch 1, wobei das gemeinsam skalierte Ultraschallbild und die virtuelle Größenbestimmungsschablone dreidimensionale Bilder sind.

4. Ultraschallsystem nach Anspruch 3, wobei das dreidimensionale Ultraschallbild und das dreidimensionale Bild der virtuellen Größenbestimmungsschablone zusammen in Reaktion auf die Benutzersteuerung geneigt und gedreht werden können.

5. Ultraschallsystem nach Anspruch 1, wobei der Bildgenerator weiterhin einen Randdetektor umfasst, der einen anatomischen Rand des anatomischen Ultraschallbilds verfolgt.

6. Ultraschallsystem nach Anspruch 1, wobei die Benutzersteuerung weiterhin durch den Benutzer bedienbar ist, um die relative Opazität oder Transparenz der Anatomie des Ultraschallbilds und der virtuellen Größenbestimmungsschablone zu verändern.

7. Ultraschallsystem nach Anspruch 1, wobei die virtuelle Größenbestimmungsschablone weiterhin ein skaliertes Bild einer implantierbaren Vorrichtung umfasst.

8. Verfahren zur Bestimmung der für die Anatomie eines Körpers geeigneten Größe einer implantierbaren Vorrichtung, umfassend:
Anzeigen eines skalierten Ultraschallbilds der Anatomie des Körpers, an der die implantierbare Vorrichtung positioniert werden soll;
Anzeigen eines gemeinsam skalierten Bilds einer virtuellen Größenbestimmungsschablone (74'), die die Größe einer implantierbaren Vorrichtung mit dem skalierten Ultraschallbild der Anatomie angibt;
Anzeigen, mit Hervorhebung, der Presspassung des skalierten Bilds der virtuellen Größenbestimmungsschablone mit dem skalierten Ultraschallbild der Anatomie; und
Manipulieren der virtuellen Größenbestimmungsschablone in Bezug auf die Anatomie des Ultraschallbilds, um zu bestimmen, ob die virtuelle Größenbestimmungsschablone korrekt zur Anatomie in dem Ultraschallbild passt, wobei das Anzeigen weiterhin das Anzeigen einer Vielzahl von virtuellen Größenbestimmungsschablonen für implantierbare Vorrichtungen von unterschiedlicher Größe umfasst; und
wobei das Manipulieren weiterhin das Auswählen und Manipulieren von einer der Vielzahl von Größenbestimmungsschablonen in Bezug auf die Anatomie des Ultraschallbilds umfasst.

9. Verfahren nach Anspruch 8, wobei das Anzeigen weiterhin das Anzeigen eines skalierten dreidimensionalen Ultraschallbilds der Anatomie des Körpers umfasst.

10. Verfahren nach Anspruch 9, wobei das Manipulieren weiterhin das Drehen oder Neigen der virtuellen Größenbestimmungsschablone und der Anatomie zusammen umfasst.

## Revendications

1. Système à ultrasons utilisé pour planifier une procédure chirurgicale avec un dispositif implantable, comprenant :
une sonde à ultrasons (14) disposée de façon à acquérir les signaux de réflexes ultrasonores d'une région volumétrique du corps, où un dispositif implantable doit être situé ;
un générateur d'image (30) sensible aux signaux de réflexes ultrasonores provenant de la sonde à ultrasons, et adapté pour agir comme une source d'images ultrasonores anatomiques mises à l'échelle du site dans un corps où un dispositif implantable doit être situé ;
une source d'une ou plusieurs images mises à l'échelle d'un calibreur (70) disposé de façon à former un calibreur virtuel (74') qui indique la taille du dispositif implantable, ladite source comprenant des images mises à l'échelle de calibreurs virtuels pour des dispositifs implantables de différentes tailles ;
un mesureur (54) sensible à l'image ultrasonore anatomique mise à l'échelle provenant du générateur d'image et à l'image mise à l'échelle du calibreur provenant de la source, ledit mesureur étant adapté pour mettre l'image du calibreur à une échelle commune correspondant à l'image ultrasonore anatomique mise à l'échelle ;
un système d'affichage (28) sensible à l'image ultrasonore anatomique et au calibreur virtuel pour mettre en évidence des zones où ledit calibreur ne s'adapte pas au site dans l'image ultrasonore anatomique où le dispositif implantable doit être situé ;
un écran (62) adapté pour afficher l'image ultrasonore anatomique communément mise à l'échelle et le calibreur virtuel ; et
une commande d'utilisateur (66) actionnable par un utilisateur pour manipuler le positionnement du calibreur virtuel sur l'écran relativement à l'anatomie de l'image ultrasonore anatomique communément mise à l'échelle, ladite commande d'utilisateur pouvant en outre être utilisée pour tenter d'adapter le calibreur virtuel dans l'anatomie de l'image ultrasonore.

2. Système à ultrasons selon la revendication 1, dans lequel les images ultrasonores sont des images ultrasonores en trois dimensions.

3. Système à ultrasons selon la revendication 1, dans lequel l'image ultrasonore communément mise à l'échelle et le calibreur virtuel sont des images en trois dimensions.

4. Système à ultrasons selon la revendication 3, dans lequel les images ultrasonores et les images du calibreur virtuel en trois dimensions peuvent être inclinées et tournées ensemble en réponse à une commande de l'utilisateur.

5. Système à ultrasons selon la revendication 1, dans lequel le générateur d'image comprend en outre un détecteur de bordure qui trace une bordure anatomique de l'image ultrasonore anatomique.

6. Système à ultrasons selon la revendication 1, dans lequel la commande d'utilisateur peut en outre être actionnée par l'utilisateur afin de modifier l'opacité ou la transparence relative de l'anatomie de l'image ultrasonore et du calibreur virtuel.

7. Système à ultrasons selon la revendication 1, dans lequel le calibreur virtuel comprend en outre une image mise à l'échelle d'un dispositif implantable.

8. Procédé de vérification de la taille d'un dispositif implantable qui est approprié pour l'anatomie d'un corps comprenant :
l'affichage d'une image ultrasonore mise à l'échelle de l'anatomie du corps où le dispositif implantable doit être situé ;
l'affichage d'une image communément mise à l'échelle d'un calibreur virtuel (74') qui indique la taille du dispositif implantable avec l'image ultrasonore mise à l'échelle de l'anatomie ;
l'affichage avec la mise en surbrillance de l'ajustement serré de l'image mise à l'échelle du calibreur virtuel avec l'image ultrasonore mise à l'échelle de l'anatomie ; et
la manipulation du calibreur virtuel relativement à l'anatomie de l'image ultrasonore afin de vérifier si le calibreur virtuel s'adapte convenablement à l'anatomie dans l'image ultrasonore, dans lequel l'affichage comprend en outre l'affichage d'une pluralité de calibreurs virtuels pour des dispositifs implantables de différentes tailles et
dans lequel la manipulation comprend en outre la sélection et la manipulation d'un parmi la pluralité de calibreurs virtuels relativement à l'anatomie de l'image ultrasonore.

9. Procédé selon la revendication 8, dans lequel l'affichage comprend en outre l'affichage d'une image ultrasonore en trois dimensions mise à l'échelle de l'anatomie du corps.

10. Procédé selon la revendication 9, dans lequel la manipulation comprend en outre la rotation ou l'inclinaison du calibreur virtuel et de l'anatomie ensemble.
